# EUROPEAN PATENT SPECIFICATION

(11) **EP 2 069 475 B1**
(45) Date of publication and mention of the grant of the patent: **18.04.2018**
(21) Application number: 07734970.2
(22) Date of filing: 26.06.2007
(51) Int. Cl.: C12N 1/00, C12N 1/20

(54) **CULTURE MEDIA FORMULATIONS FOR INDUSTRIAL APPLICATION**
KULTURMEDIENFORMULIERUNGEN FÜR DIE GROSSTECHNISCHE ANWENDUNG
PRÉPARATIONS DE MILIEUX DE CULTURE POUR APPLICATIONS INDUSTRIELLES

(30) Priority: 27.09.2006 IT MI20061843
(43) Date of publication of application: 17.06.2009
(73) Proprietor: Mofin S.r.l., 28100 Novara (IT)
(72) Inventor: MOGNA, Giovanni, 28100 Novara (IT); BRUNO, Federico, 28060 Granozzo con Monticello Frazione Case Sparse (IT)
(74) Representative: Hoffmann Eitle
(86) International application number: PCT/IB2007/001911
(87) International publication number: WO 2008/038076

(56) References cited:
- EP-A- 0 575 951
- WO-A-00/39281
- WO-A-02/46370
- WO-A-84/04106
- WO-A-2005/045050
- WO-A-2006/013588
- WO-A-2006/067136
- DE-A1- 2 912 778
- US-A- 2 792 389
- US-A- 4 382 965
- US-A- 4 956 295
- US-A1- 2004 001 814
- US-B1- 7 037 708
- CHAPMAN ET AL: "Improved methods for the cultivation of the chemolithoautotrophic bacterium Nitrosomonas europaea" JOURNAL OF MICROBIOLOGICAL METHODS, ELSEVIER, AMSTERDAM,, NL, vol. 65, no. 1, April 2006 (2006-04), pages 96-106, XP005340207 ISSN: 0167-7012
- ESCAMILLA-HURTADO ET AL: "Effect of culture conditions on production of butter flavor compounds by Pediococcus pentosaceus and Lactobacillus acidophilus in semisolid maize-based cultures" INTERNATIONAL JOURNAL OF FOOD MICROBIOLOGY, ELSEVIER SCIENCE PUBLISHERS, AMSTERDAM, NL, vol. 105, no. 3, 15 December 2005 (2005-12-15), pages 305-316, XP005186599 ISSN: 0168-1605
- WANG R ET AL: "Protease production and conidiation by Aspergillus oryzae in flour fermentation" PROCESS BIOCHEMISTRY, ELSEVIER, NL, vol. 40, no. 1, January 2005 (2005-01), pages 217-227, XP004558545 ISSN: 1359-5113
- CHAMPAGNE ET AL: "Fresh-cheesemilk formulation fermented by a combination of freeze-dried citrate-positive cultures and exopolysaccharide-producing lactobacilli with liquid lactococcal starters" FOOD RESEARCH INTERNATIONAL, ELSEVIER APPLIED SCIENCE, BARKING, GB, vol. 39, no. 6, July 2006 (2006-07), pages 651-659, XP005365860 ISSN: 0963-9969
- CHAMPAGNE C P ET AL: "The potential of immobilized cell technology to produce freeze-dried, phage-protected cultures of Lactococcus lactis" FOOD RESEARCH INTERNATIONAL, vol. 25, no. 6, 1992, pages 419-427, XP002455800 ISSN: 0963-9969
- NICOLAS G ET AL: "Improved methods for mutacin detection and production" JOURNAL OF MICROBIOLOGICAL METHODS, ELSEVIER, AMSTERDAM,, NL, vol. 59, no. 3, December 2004 (2004-12), pages 351-361, XP004607236 ISSN: 0167-7012
- THARMARAJ N ET AL: "Survival of Lactobacillus acidophilus, Lactobacillus paracasei subsp. paracasei, Lactobacillus rhamnosus, Bifidobacterium animalis and Propionibacterium in cheese-based dips and the suitability of dips as effective carriers of probiotic bacteria" INTERNATIONAL DAIRY JOURNAL, ELSEVIER APPLIED SCIENCE, BARKING,, GB, vol. 14, no. 12, December 2004 (2004-12), pages 1055-1066, XP004573490 ISSN: 0958-6946
- BOCKELMANN W ET AL: "Cultures for the ripening of smear cheeses" INTERNATIONAL DAIRY JOURNAL, ELSEVIER APPLIED SCIENCE, BARKING,, GB, vol. 15, no. 6-9, June 2005 (2005-06), pages 719-732, XP004910520 ISSN: 0958-6946
- VIZOSO PINTO MARIA G ET AL: "Lactobacillus spp. with in vitro probiotic properties from human faeces and traditional fermented products" INTERNATIONAL JOURNAL OF FOOD MICROBIOLOGY, ELSEVIER SCIENCE PUBLISHERS, AMSTERDAM, NL, vol. 109, no. 3, 28 February 2006 (2006-02-28), pages 205-214, XP002398656 ISSN: 0168-1605

## Description

The present invention relates to formulations of culture mediums for the industrial development of liquid starter cultures characterized by a larger number of microbial cells per volume unit of fermentation medium than the one of traditional liquid starter cultures, which number can be defined a priori depending on the formulation of said medium.

Cultures of microorganisms added to several types of food are referred to as "starter cultures". Said starter cultures are selected grafts, made up of microbial strains with well defined biochemical properties, which therefore enable to obtain repeatable and time-constant results.

In all biotechnological applications, the role of starter cultures is to start and correctly direct fermentative processes, thus contributing significantly to the achievement not only of sensorial but often also of structural peculiarities of finished products.

Said microbial starter cultures are now universally used in the preparation of the most different food products and the quality thereof is related not only to a correct technological practice but also to the functional specificity of the culture used and to the number and physiological state of the microorganisms making up said culture.

Starter cultures can be classified on the basis of various criteria, depending on the complexity of the composition (cultures made up of only one microbial strain or of more strains belonging to one or more species and/or genus), on the field of application (e.g. dairy field or others) and on the physical state of the culture (liquid, frozen or dehydrated, e.g. by freeze-drying or spray-drying).

Referring to the physical form, liquid cultures, if prepared correctly and used within a short lapse of time, are those giving the best results, since they are characterized by a microbial population with a perfect physiological state and can therefore replicate and manifest their role straight after inoculation into the food or starting materials to be transformed.

Negative aspects of liquid cultures, which constitute at present a limiting factor to their diffusion, consist in the short shelf-life, which can reach a maximum of 4-5 days (at a storage temperature of 3-5°C) and in the limited maximum cell concentration that can be obtained (which forces to use large volumes of said cultures, the amount of starting material to be transformed being the same).

Conversely, freeze-dried and frozen cultures are characterized by far longer shelf-lives and by smaller volumes.

However, freeze-dried and frozen cultures have the serious drawback consisting in that cells lie in a physiological state that is unable to adapt in short times to the culture conditions of the food substrate to be fermented. In other words, they are characterized by a more or less longer latency stage, referred to as LAG stage, which is essential for restoring vital functions.

Moreover, products (e.g. dairy products) obtained with said cultures have sensorial, aroma and taste characteristics that are on the whole inferior with respect to products prepared by the application of liquid starter cultures.

Therefore, there is still the need for liquid cultures having a higher cell concentration, which are thus sufficient for transforming larger amounts of starting materials and with lower costs, ensuring the industrial production of products with higher organoleptic properties.

Unfortunately, culture mediums (referred to as conventional) that are commonly used for the industrial production of known starter cultures (referred to as traditional) cannot give an adequate answer to the need disclosed above.

Said conventional commercial mediums are commonly made up of proteins, proteides and/or peptones, of simple or complex glucides, of vitamins, mineral salts and specific growth factors for each microbial genus and species.

Nitrogen sources bring in the material required for the construction of cell structures, whereas carbon sources supply the energy required for the various metabolic transformations.

WO 00/39281 relates to a method for retaining the initial metabolic activity of a liquid starter culture by addition of stabilizing compounds, including formic acid, formate and compounds involved in the biosynthesis of nucleic acids.

WO 2006/067136 is directed to a method for the preparation of a starter culture for the production of fermented dairy products in the presence of a stimulant such as yeast extracts, proteins or nucleotides in a pre-pasteurised medium. Preparation of starter cultures of Streptococcus thermophilus strains is described in example 4 in presence of external pH control (injection of ammonia) to maintain the pH above 5.8. At present, the limiting factor to the industrial achievement of liquid cultures with high bacterial concentration consists in the pH decrease of the culture medium during the production of the microbial biomass; said phenomenon is due to the production of organic acids through fermentation of the carbon sources during bacterial reproduction.

Organic acids, in particular lactic and acetic acid, when in dissociated form, are toxic for bacterial cells, and if the concentration thereof is above a given threshold, varying from species to species, they sufferance and then block the metabolic activities. If the incubation stage of the culture in the culture medium goes on for an excessively long time, the result is a high mortality in the bacterial population, compromising the function thereof.

There are basically two consequences of this phenomenon: on the one hand there is the loss, more or less evident, of vitality of the biomass, on the other hand there is a phenomenon of inhibition of the further development of said biomass, which obviously negatively affects cell concentration and the global fermentative activity of the final starter culture.

The microbial load (or microbial cell concentration per volume unit of final starter culture, expressed as CFUs, colony-forming units, per ml of final starter culture) that can be obtained with culture mediums known at present and used industrially (conventional mediums) is of max. 0.5-1•10⁹ CFUs/ml, but it is often not above 200 millions (2**•**10⁸) CFUs/ml.

In the dairy field, the necessary amount of liquid starter culture varies from one product to another, depending on the inoculation envisaged for each type of cheese-making process and on the cell density of the culture.

In general, inoculation enables to obtain in milk a concentration of vital cells of at least 10-25 millions cells/ml. Since a traditional starter culture contains maximum 0.5•10⁹-1•10⁹ CFUs/ml, inoculation represents in volume 1% to 5% of the total milk volume; therefore, 10-50 liters of traditional starter culture will be required for inoculating 1,000 liters of milk and then achieving the necessary fermentative activity, which can be measured by way of pH decrease in milk as a function of time.

The previous example has confirmed that the volumes involved are quite high and, therefore, a high volume of liquid starter culture is sufficient to transform a limited volume of starting material: it is thus necessary for the industry to frequently produce further starter culture, which results in a lower plant productivity and sometimes unbearable costs.

In the light of the problems and of the need disclosed above, it would therefore be particularly useful to have a culture medium that enables to produce liquid starter cultures characterized by a high cell concentration (therefore, by limited volumes) and by a high fermentative activity, and maintaining at the same time the favorable properties of known liquid starter cultures (especially in terms of better organoleptic properties of the products obtained from their use), as well as the easier global management characterizing frozen or dehydrated cultures.

Culture mediums for the industrial development of the microorganism/s of a liquid starter culture having the properties referred to above are not known at present.

There is still the need, therefore, for culture mediums that enable to produce liquid starter cultures having the advantageous properties disclosed above.

The aim of the present invention is to answer adequately the need referred to above.

This aim and others, which shall be apparent from the following detailed description, have been achieved by the Applicant, who has unexpectedly found that the addition of a suitable amount of at least one basic neutralizing agent directly into the starting culture medium used for growing the microbial biomass enables to obtain a liquid starter culture characterized by a considerable increase in the number of microbial cells with respect to liquid starter cultures known at present (traditional cultures).

Therefore, an object of the present invention is a method for preparing a liquid starter culture including addition of the above culture medium, as disclosed in the appended independent claim.

Another object of the present invention is the use of said medium for the industrial production of liquid starter cultures, especially for the cheese-making field, as disclosed in appended independent claim. In another aspect the present invention provides a liquid starter culture with high cell concentration and high fermentative activity, as can be obtained with said culture medium, whose characteristics are disclosed in the appended independent claim.

Preferred embodiments of the present invention are disclosed in the appended dependent claims.

The term microbial cell concentration refers to the number of vital microbial cells (measured as colony-forming units or CFUs) per volume unit of culture or culture medium.

Cell concentration is determined by way of one or more vital counts, generally on plates. Such method consists in determining the number of cells present in a fermentation medium that are able to form colonies on lab plates containing an adequate volume (generally 10 ml) of an agar medium. The fermentative activity of a liquid starter culture is determined by measuring pH decrease in time of a given volume of a suitable liquid, preferably milk, after inoculation with a given volume of said culture.

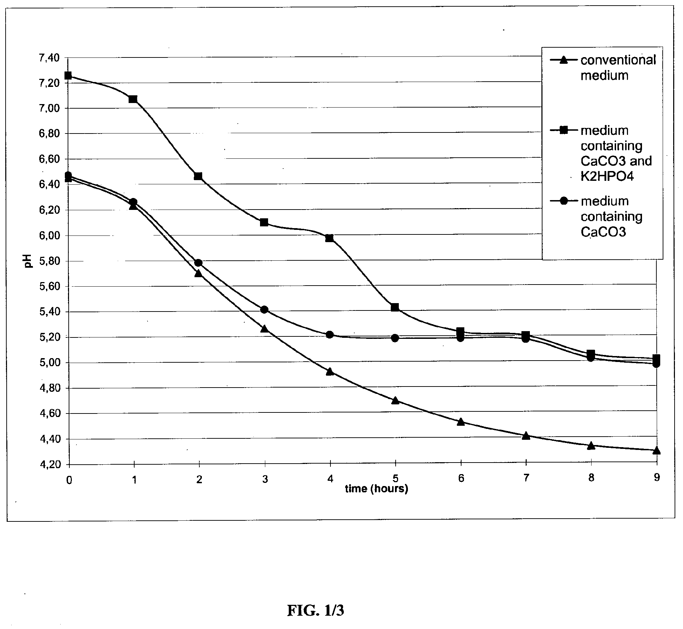

The characteristics and advantages of the present invention are pointed out in the following detailed description; moreover, they are further disclosed by way of example also in the accompanying **Fig. 1-3** and in the accompanying **Tables 1-3** related to said figures, in which:
- **Fig. 1** is a graph of the development in time of: pH of a conventional culture medium during the industrial production of a traditional liquid starter culture, pH of a culture medium containing calcium carbonate as inner neutralizing agent during the industrial production of a liquid starter culture according to the present invention; pH of a culture medium containing calcium carbonate and potassium monohydrogenphosphate as mixture of inner neutralizing agents, during the industrial production of a liquid starter culture according to the present invention; measurements have been carried out up to the end of the cooling stage of said cultures;

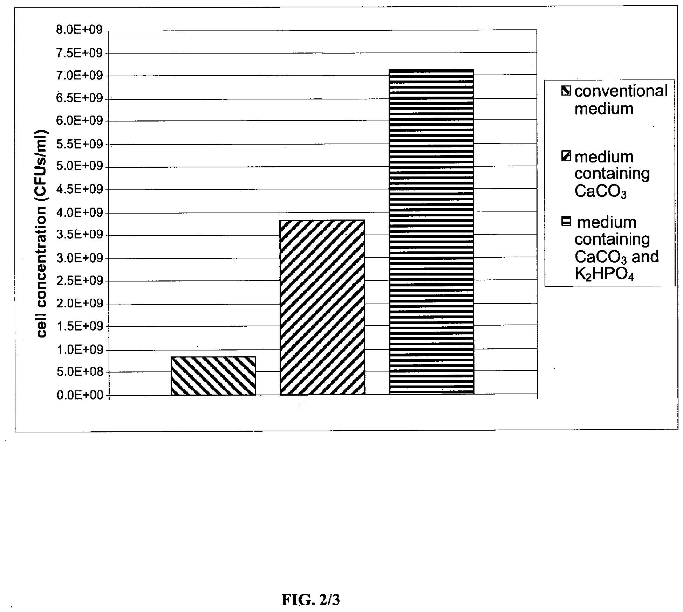

- **Fig. 2** contains the values of cell concentrations, respectively, of: a traditional liquid culture developed with a conventional culture medium: a liquid starter culture according to the invention, developed using a culture medium containing CaCO₃ as inner neutralizing agent; a liquid starter culture according to the invention, developed using a culture medium containing CaCO₃ and potassium monohydrogenphosphate as mixture of inner neutralizing agents;
   said concentration values are expressed as CFUs/ml (the term nE+p on the ordinate corresponds to n•10^{p} CFUs/ml);

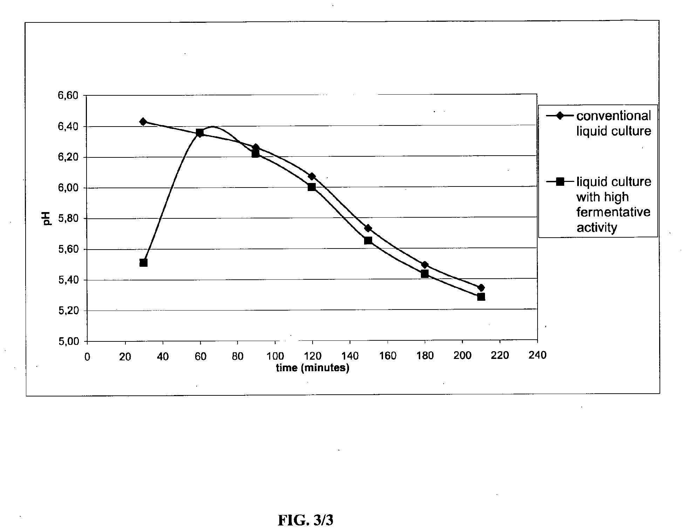

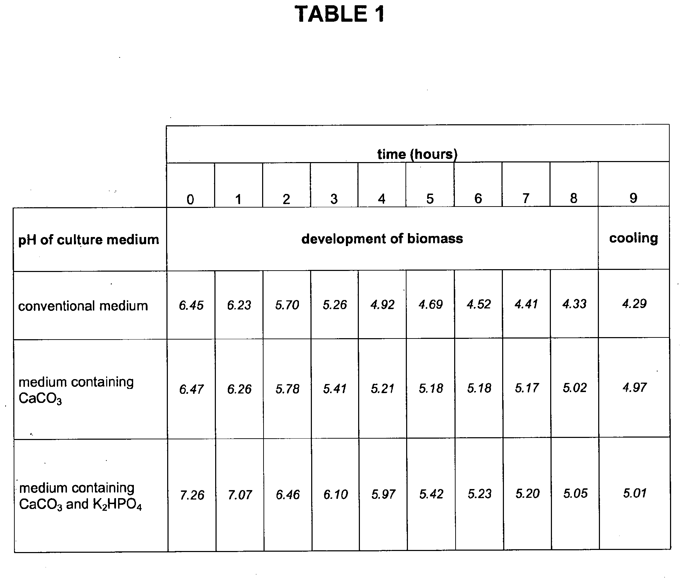

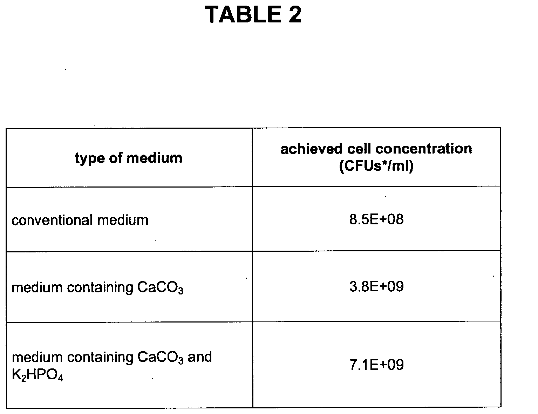

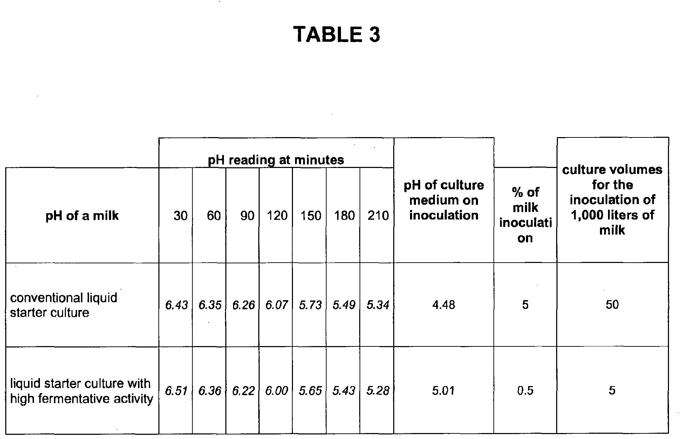

- **Fig. 3** is a graph of the fermentative activity shown, during biotechnological use, by: a traditional liquid starter culture, developed with a conventional culture medium; a liquid starter culture with high fermentative activity according to the invention, developed using a culture medium containing CaCO₃ and potassium monohydrogenphosphate as inner neutralizing agents; said activities are expressed by showing the time development of pH decrease of a milk inoculated with said cultures; the traditional liquid starter culture has been inoculated to 5% of total milk volume (V/V), whereas the liquid starter culture with high fermentative activity according to the invention has been inoculated to 0.5% by volume, i.e. 10 times less; the graph clearly points out that the liquid starter culture according to the invention shows the same industrial yield as a conventional culture, using volumes that are 10 times lower (therefore, if the dose is the same, it is characterized by a fermentative activity that is 10 times higher);
- **Table 1** shows the pH values of the various production stages that resulted in the graphs of Fig. 1;
- Table 2 shows the values of cell concentration, expressed as CFUs/ml, that resulted in the histogram of Fig. 2;
- Table 3 shows the pH values that resulted in Fig. 3. The present invention relates to a culture medium, characterized in that it comprises an effective amount of at least one basic neutralizing agent.

Preferably, said culture medium is used for the industrial production of a liquid starter culture with high fermentative activity, comprising at least one physiologically compatible microorganism, wherein said liquid starter culture is characterized in that it has a higher bacterial cell concentration of said at least one microorganism than the maximum cell concentration of traditional liquid starter cultures (i.e. >10⁹ CFUs/ml of culture).

Preferably, said above liquid starter culture is a culture with direct inoculation.

Generally, a basic neutralizing agent can be chosen from the group comprising: carbonate ion, in mono- and dibasic forms, phosphate ion, in mono-, di- and tribasic forms, sulfate ion, in mono- and dibasic forms, hydroxide ion, citrate ion, in mono-, di- and tri basic forms, tartrate ion, in mono- and dibasic forms, other bases that are physiologically compatible with the microorganisms of the microbial culture to be developed in said medium, and/or mixtures thereof. Said bases derive from suitable salts in which the cationic moiety is preferably represented by calcium, sodium, potassium, magnesium, manganese and/or ammonium ion.

In the present invention, said base is calcium carbonate and potassium monohydrogenphosphate.

Advantageously, in said mixture calcium carbonate and potassium monohydrogenphosphate are in a mutual ratio (weight/weight) of 1:9 to 9:1, preferably of 1:4 to 4:1; most preferably of 1:2 to 2:1.

Said at least one neutralizing agent is advantageously used in suitable percentages (weight/weight), depending basically on the characteristics of the microbial strain/s to be developed and on the final cell concentration to be achieved.

As a matter of fact, the Applicant has unexpectedly found that the cell concentration that can be achieved at the end of the process of industrial preparation of the starter culture is directly proportional, within precise and well defined ranges, to the amount of neutralizing agent present in said medium.

Therefore, medium formulation can be defined as a function of the expected industrial results, obtaining with the same composition reproducible and constant results that can be established a priori.

The Applicant has found that the use of an inner neutralizing agents, or of a mixture thereof, enables to keep the pH of the culture medium within a precise range depending on the specific pK_{b} value/s of the base/s released with the dissolution of said agent/s.

This maintenance begins spontaneously when the pH of the medium for growing the biomass achieves specific values, as a function of the pK_{b} value of the neutralizing agent or of the mixture of agents present therein, and thus depends on the growing microorganism/s and on the culture conditions The duration of said maintenance has proved to depend both on the concentration of the neutralizing agent or mixture of agents and on the acidifying ability of the strain/s present in the bioreactor.

The total amount of the neutralizing agent is such as to allow during the pH maintenance stage, during the stage of exponential growth of the microbial biomass in the culture medium, 2 to 5 cell divisions (duplications) of the microbial biomass to occur. The total amount of neutralizing agent (or mixture of neutralizing agents) is as a rule of 2 g/1 of culture medium. Preferably, said amount is of 2 g/1 to 40 g/1; more preferably, it is of 8 g/1 to 25 g/1.

The range within which the pH of the culture medium is kept during the stage of exponential growth of the microbial biomass in said medium, is between 5.0 and 5.7.

In a preferred embodiment, which uses CaCO₃ and K₂HPO₄ as mixture of inner neutralizing agents, pH development during the maintenance stage is almost constant within the ranges 6,1-6,2 and 5,1-5,2, up to the exhaustion of said mixture.

By way of example, in a first particularly preferred embodiment of the invention, the amount of neutralizing agent, or mixture of agents, to be introduced into the formulation of the culture medium is calculated so that, once said neutralizing agent or mixture is completely exhausted, there can be such a residual amount of energy sources as to enable further 0.5-1 cell duplications of the biomass. Under such conditions, the pH of the culture medium sinks to a specific value varying as a function of the grown microorganism, said value however never being such as to cause the microbial biomass to suffer.

Under these circumstances, the fermentative activity of the culture is directly related and proportional to cell concentration, often also slightly higher since the microbial biomass lies in a perfect state of vitality and integrity.

In a second particularly preferred embodiment of the invention, the amount of neutralizing agent/s is calculated so that, after said agent/s has/have completely exhausted, there is such a residual amount of energy sources as to enable further 1 to 3 cell duplications of the bacterial biomass. Under such circumstances, said biomass has a cell concentration up to 4 times higher than the one achievable in the previous embodiment, but the fermentative activity manifested by said biomass may not be directly proportional to such factor, since these cells are not in a perfect physiological state due to the toxicity caused by the excessive hydrogen ion concentration in the final stages of industrial production of the starter culture.

The method for preparing a culture medium according to the present invention includes the addition of a suitable amount of at least one basic neutralizing agent, in accordance with the above description, preferably to any traditional culture medium (e.g. commercially available), depending on the microorganism/s to be developed.

Preferably, said addition is carried out by traditional mixing of the components in a suitable mixing apparatus.

Said addition/mixing can be carried out in dry conditions (mixing of powders of the components) or in liquid phase (e.g. under stirring), after diluting medium components and neutralizing agent/s in a suitable amount of a liquid, preferably aqueous medium.

The pH value of the concentrated liquid starter culture with high fermentative activity, obtained from the medium according to the invention, is generally between 4.7 and 5.6, preferably between 4.9 and 5.2.

Now the final culture containing water, the microbial biomass, the metabolites produced by said biomass during the growth stage, and the residues of components of the initial culture medium, is cooled to a temperature of 4°C to 10°C and stored under refrigerated conditions (preferably at 3°C to 6°C) up to the biotechnological application of said culture. Therefore, an object of the present invention is also the liquid starter culture obtained by using the culture medium of the invention as described above.

In an embodiment of the invention, the microorganism or mixture of microorganisms of the starter culture is chosen among suitable, physiologically compatible microbial strains.

In another embodiment of the invention, said microorganism/s is/are chosen among microbial strains having a probiotic valence.

The cell concentration of said liquid starter culture, obtained using the culture medium according to the present invention, is as a rule >1.5 times as much as the one of traditional liquid starter cultures.

Preferably, said liquid culture has a cell concentration that is ≥2.5 times as much as the concentration of traditional liquid starter cultures; more preferably, said concentration is ≥5 times as much as the one of traditional liquid starter cultures.

For instance, the cell concentration of the liquid starter culture, obtained in accordance with the procedure described in the above first particularly preferred embodiment of the invention, is as a rule ≥1.5 times as much as the one of traditional liquid starter cultures.

Said liquid culture has a cell concentration that is preferably 2.5 to 15 times as much as the concentration of traditional liquid starter cultures; more preferably, said concentration is 4 to 12 times as much as the one of traditional liquid starter cultures.

Still more preferably, said concentration is ≥5 times as much as the one of traditional liquid starter cultures.

In its turn, the cell concentration of the liquid starter culture, obtained in accordance with the procedure described in the above second particularly preferred embodiment of the invention, is as a rule 2.5 to 60 times as much as the concentration of known liquid starter cultures; preferably, said concentration is 8 to 32 times as much as the concentration of known liquid starter cultures.

More preferably, said concentration is ≥16 times as much as the one of known liquid starter cultures. Therefore, the liquid starter culture according to the present invention is characterized by a cell concentration of >10⁹ CFUs/ml of culture; preferably, >1.5•10⁹ CFUs/ml of culture.

In a preferred embodiment of the invention, the above liquid starter culture is characterized by a cell concentration of ≥2.5•10⁹ CFUs/ml of culture, preferably, ≥5•10⁹ CFUs/ml.

Advantageously, the liquid starter culture according to the present invention has a higher fermentative activity than the one of known liquid starter cultures.

Said fermentative activity has proved to be on average at least 2 times, preferably at least 2.5 times as much as the one of known liquid starter cultures.

For instance, in the above first particularly preferred embodiment of the invention, said liquid starter culture has a fermentative activity 2.5 to 18 times as much as the fermentative activity of traditional liquid starter cultures; preferably, said activity is 4 to 15 times as much. More preferably, said fermentative activity is ≥6 times as much as the activity of traditional liquid starter cultures.

In its turn, in the above second particularly preferred embodiment of the invention, said liquid starter culture has a fermentative activity 2.5 to 30 times as much as the fermentative activity of traditional liquid starter cultures; preferably, said activity is 8 to 24 times as much as the fermentative activity of traditional liquid starter cultures.

More preferably, said fermentative activity is ≥16 times as much as the activity of traditional liquid starter cultures.

The liquid starter culture with high concentration and high fermentative activity, obtained with the culture medium according to the present invention, is a ready-to-use culture for any industrial application.

Quite unexpectedly, said liquid starter culture has also proved to be more stable than traditional liquid starter cultures. As a rule, the above liquid starter culture has proved to have a storability that is ≥1.5 times as much as the storability of traditional liquid starter cultures.

Preferably, said storability is ≥2.5 times as much. More preferably, said storability is ≥4 times as much. The liquid starter culture according to the present invention, therefore, is characterized by a storability of ≥6 days, at an average storage temperature of 3°C to 5°C. Preferably, said storability is of ≥7.5 days; more preferably of ≥10 days, still more preferably, it is of ≥13 days.

Conventionally, in the present invention storability is always evaluated referring to an average storage temperature of 3°C to 5°C.

Thanks to the addition of at least one inner basic neutralizing agent, preferably calcium carbonate and/or a mixture of said carbonate with at least another one suitable salt as defined above, it has been possible to obtain in the starting culture medium, in a reproducible and a-priori definable manner, a higher development of the biomass, a higher vitality and an increased fermentative activity of the resulting culture, as well as an unexpected increased stability of said culture.

The method for preparing a liquid starter culture according to the present invention includes at least one stage in which a culture medium, preferably of conventional type, is added/mixed with a suitable amount of at least one basic neutralizing agent in accordance with the above description.

As an absolutely non-limiting example of embodiment, the following discloses a general method for preparing a liquid starter culture with high concentration and high fermentative activity, wherein said culture is obtained by using a culture medium according to the present invention as described above. Said method basically includes the following steps:
a) adding to a culture medium a suitable amount of at least one basic neutralizing agent;
b) dissolving the medium from a) in a suitable volume of a liquid medium, usually water;
c) decontaminating the bioreactor by flowing vapor; preferably for 30 minutes;
d) thermally treating the diluted culture medium from b) in the bioreactor; preferably at 85C°-90°C for 20-30 minutes;
e) cooling the culture medium from d) up to the desired temperature of inoculation;
f) inoculating the culture medium from e) with an effective amount of mother culture (liquid, freeze-dried or frozen) of at least one microbial bacterial microorganism, or of a mixture of microorganisms;
g) letting the biomass develop for a time sufficient for a total of 8 to 10 cell duplications of the microorganism/s to be grown to occur, until - once the buffering effect of the neutralizing agent or agents is over - pH of the culture medium spontaneously links to a value of 4.9 to 5.2; advantageously, during the whole period of development of the biomass, the fermentation medium is kept under slight and constant stirring;
h) cooling the culture from g) up to a temperature of 4°C to 8°C.

In a preferred example of embodiment of the invention, absolutely non-limiting, the culture medium from b), containing calcium carbonate as inner neutralizing agent, comprises (amounts referred to one liter of culture medium): serum, 5 g; casein peptone 10 g; yeast extract, 5 g; glucose, 10 g; MnSO₄, 200 mg; CaCO₃, 12 g; water, q.s. to 1 1 of final culture medium.

The amount of calcium carbonate present in said culture medium is generally of 2 g/1 of culture medium. Preferably, said concentration is of 2 to 20 g/1, more preferably, it is of 5 to 15 g/1; still more preferably, of 8 to 12 g/l.

In another preferred embodiment, absolutely non-limiting, the culture medium from b), containing calcium carbonate and potassium monohydrogenphosphate as mixture of inner neutralizing agents, comprises (amounts referred to one liter of culture medium): serum, 5 g; casein peptone 10 g; yeast extract, 5 g; glucose, 10 g; MnSO₄, 200 mg; CaCO₃, 4.8 g; K₂HPO₄, 12.1 g ; water, q.s. to 1 1 of final culture medium.

The total amount of calcium carbonate and of potassium monohydrogenphosphate is
of 8 to 20 g/1.

Advantageously, the presence of calcium carbonate, either alone or mixed with at least another salt, results in the development of carbon dioxide in the Fermentation medium, when the pH of said medium sinks below well defined thresholds depending on the pK_{b} values of carbonate ion.

The Applicant has unexpectedly found that the development of CO₂ by carbonate ion works as bioactivator towards growing microorganisms and also contributes to the anaerobiosis of the medium for the development of the biomass, resulting in huge advantages when said biomass is made up of strictly anaerobic microorganisms or microaerophilic microorganisms.

The inoculation temperature of the culture medium from e) is on average of 18°C to 47°C, depending on the characteristics of the microorganism/s used, on the corresponding growth conditions required by said microorganism/s, and on the percentage of inoculation of the microorganism/s. For instance, for mesophilic microorganisms said temperature is preferably of about 18°C to about 32°C, whereas for thermophilic microorganisms, said temperature is preferably of about 37°C to about 45°C.

The amount of mother culture as in step f), added to the culture medium from b), if liquid or frozen, can vary on average from 0.5% to 10% (V/V), depending on the microorganism/s to be grown, on the characteristics of the culture medium and on the conditions of development of the microbial biomass.

In case of dehydrated mother cultures, the amount is function of bacterial concentration and is anyhow such as to give on average 10-25 millions CFUs/ml of culture medium.

Preferably, measures apt to prevent as much as possible contamination risks are taken for the bioreactor: air getting into the fermentation compartment is filtered through sterile filters; the compartment itself is sterilized with high-temperature vapor between one production cycle and the following one; also electrodes used for pH control and temperature sensors are sterilized; biomass production takes place under sterile air or nitrogen overpressure conditions.

The liquid starter culture obtainable by using the culture medium according to the present Invention, as described above, can comprise any microbial bacterial strain that is physiologically compatible and/or is interesting in terms of industrial application. Preferably, said culture comprises a mixture of more microbial strains chosen from the group comprising the genus:
*Lactobacillus, Bifidobacterium, Lactococcus, Leuconostoc, Pediococcus, Streptococcus, Bacillus, Propionibacterium, Saccharomyces, Enterococcus, Staphylococcus.*

For instance, the following species of the genus *Lactobacillus* have been used: L. *pentosus, L. plantarum, L. casei, L. casei ssp. paracasei, L. casei ssp. rhamnosus, L. acidophilus, L. delbrueckii ssp. bulgaricus, L. fermentum,* L. *gasseri.*

For instance, the following species of the genus *Bifidobacterium* have been used: *B. longum, B. breve, B. lactis, B. adolescentis, B. pseudocatenulatum, B. catenulatum.*

For instance, the following species of the genus *Lactococcus* have been used: *L*. *lactis* and *L*. *lactis ssp. lactis.* For instance, the species *S. thermophilus* of the genus *Streptococcus* has been used

For instance, the species *S. xylosus* of the genus *Staphylococcus* has been used.

With the embodiments of the present invention, both cell concentration and the fermentative activity of the bacterial population of the starter culture thus obtained can be defined a priori, as a function of the amount of neutralizing agent or mixture thereof present in the medium.

Such possibility is even more advantageous if food ripening has to be anticipated.

As a matter of fact, the Applicant has unexpectedly found that a suitable lower fermentative activity of the bacterial biomass, the cell concentration obtainable with a culture medium in accordance with the second preferred embodiment of the invention being the same, enables to introduce into the starting material to be transformed - if this is advantageous in technological and industrial terms - a higher amount of intracellular enzymes (which mediate the proteolytic/lipolytic activity of the starting materials, therefore the subsequent aroma development) than the one that would be introduced by a culture characterized by a higher fermentative activity.

The liquid starter cultures according to embodiments of the invention are used as grafts for preparing industrial food products (e.g. dairy products such as cheese, yogurts, fermented milks; bread, baked products, salamis and sausages in general, alcoholic drinks).

Preferably, said liquid starter cultures are used for preparing industrial dairy products.

The cheese-making process to be followed with starter cultures having a high fermentative activity according to the invention is the same as the one with traditional liquid starter cultures; the dairy products thus obtained have at least all the desirable organoleptic properties that can be obtained with said traditional liquid starter cultures.

The inoculation of milk in a fermenter with a liquid starter culture having a high fermentative activity is on average below 1% by volume (V/V) with respect to the total volume of milk to be treated; preferably, said inoculation is of 0.2% to 0.8% (V/V) with respect to milk; more preferably, it is of 0.3% to 0.6% (V/V); advantageously, it is of ≤0.5% (V/V).

Therefore, the volume of milk to be inoculated being the same, the use of a smaller volume of liquid starter culture with high fermentative activity is sufficient. For the inoculation of 1,000 liters of milk, for instance, 3 to 6 liters of starter culture according to the present invention are sufficient, instead of 10-50 liters required for the inoculations of the same amount of milk with a known liquid starter culture. Such volume of liquid starter culture with high fermentative activity corresponds - in terms of industrial yield, which can be measured by way of the decrease of pH values in milk as a function of time - to 10-50 liters of a known commercial liquid starter culture.

As an absolutely non-limiting example, the following discloses the composition of the culture medium according to the present invention, with which two liquid starter cultures with high fermentative activity have been developed.

Example 1 - Culture medium for the production of a liquid starter culture comprising the bacterial strain *Streptococcus thermophilus* DSM 16506 (deposited by ANIDRAL S.r.l. in DSMZ on 18.06.2004).

Said medium has the following composition (amounts referred to one liter of culture medium): serum permeate, 8 g; casein peptone, 12 g; yeast extract, 5 g; CaCO₃, 9 g; water, q.s. to 1 1 of culture medium.

Following the procedure of the general method of preparation described above, by way of the above medium a liquid starter culture for dairy use has been obtained, having a final cell concentration of *Streptococcus thermophilus* DSM 16506 corresponding to 3.8 10⁹ CFUs/ml of starter culture.

Example 2 - Culture medium for the production of a liquid starter culture comprising the bacterial strain *Streptococcus thermophilus* DSM 16506.

Said medium has the following composition (amounts referred to one liter of culture medium): serum permeate, 8 g; casein peptone, 12 g; yeast extract, 5 g; CaCO₃, 3.6 g; K₂HPO₄, 9.1 g; water, q.s. to 1 1 of final culture medium.

Following the procedure of the general method of preparation described above, by way of the above medium a liquid starter culture for dairy use has been obtained, having a final cell concentration of *Streptococcus thermophilus* DSM 16506 corresponding to 7.1 10⁹ CFUs/ml of culture.

## Claims

1. A method for preparing a liquid starter culture comprising at least one microorganism selected from the group of microbial strains of the species *Streptococcus thermophilus* having a microbial cell concentration of said at least one microorganism of more than 1 x 10⁹ CFUs/ml,
wherein said method includes at least a step in which a culture medium is added with an effective amount of at least one basic neutralizing agent containing a mixture of calcium carbonate and potassium monohydrogenphosphate, wherein said neutralizing agent is present in such an amount as to enable to keep the pH value of said medium within a given range, during the stage of exponential growth of the microbial biomass in said medium,
wherein said given pH range is 5.0 to 5.7, and
wherein the concentration of the total amount of calcium carbonate and of potassium monohydrogenphosphate in the culture is of 8 g/l to 20 g/l.

2. The method according to claim 1, including the following steps:
a) adding to a culture medium an amount of at least one basic neutralizing agent;
b) dissolving the medium from a) in a liquid medium, preferably water;
c) decontaminating the bioreactor by flowing vapor; preferably for 30 minutes;
d) thermally treating the diluted culture medium from b) in the bioreactor; preferably at 85°C-90°C for 20-30 minutes;
e) cooling the culture medium from d) up to the temperature of inoculation;
f) inoculating the culture medium from e) with an effective amount of mother culture of at least one microbial bacterial microorganism, or of a mixture of microorganisms;
g) letting the biomass develop for a time sufficient for a total of 8 to 10 cell duplications of the microorganism/s to occur, until the pH of the culture medium spontaneously sinks to a value of 4.9 to 5.2;
h) cooling the culture from g) up to a temperature of 4°C to 8°C.

3. The method according to any one of claims 1 to 2, wherein the bacterial strain is *Streptococcus thermophilus* DSM 16506.

4. Use of a liquid starter culture prepared according to the method of any one of the claims 1 to 3 as starter for preparing industrial food products.

5. Use according to claim 4, wherein said industrial food products are chosen from the group comprising: dairy products, cheese, yogurt, fermented milks, bread, baked products, salamis, fermented sausages, alcoholic drinks.

6. Use according to claim 5, wherein said products are chosen among dairy products.

7. Use of a liquid starter culture prepared according to the method of any one of the claims 1 to 3 for direct milk inoculation.

## Patentansprüche

1. Verfahren zur Herstellung einer flüssigen Starterkultur, umfassend wenigstens einen Mikroorganismus, ausgewählt aus der Gruppe von Mikrobenstämmen der Spezies *Streptococcus thermophilus* mit einer mikrobiellen Zellkonzentration dieses wenigstens einen Mikroorganismus von mehr als 1 x 10⁹ CFUs/ml,
wobei das Verfahren wenigstens einen Schritt einschließt, in dem ein Kulturmedium mit einer wirksamen Menge wenigstens eines basischen Neutralisierungsmittels versetzt wird, das eine Mischung aus Calciumcarbonat und Kaliummonohydrogenphosphat enthält,
wobei das Neutralisierungsmittel in einer Menge vorliegt, die es ermöglicht, den pH-Wert während des exponentiellen Wachstumsstadiums der mikrobiellen Biomasse in dem Medium innerhalb eines gegebenen Bereiches zu halten,
wobei dieser gegebene pH-Bereich 5,0 bis 5,7 beträgt, und
wobei die Konzentration der Gesamtmenge von Calciumcarbonat und von Kaliummonohydrogenphosphat in der Kultur 8 g/l bis 20 g/l beträgt.

2. Verfahren gemäß Anspruch 1, welches die folgenden Schritte einschließt:
a) Zugabe einer Menge wenigstens eines basischen Neutralisierungsmittels zu einem Kulturmedium;
b) Auflösen des Mediums aus a) in einem flüssigen Medium, vorzugsweise Wasser;
c) Dekontaminieren des Bioreaktors durch Einblasen von Wasserdampf, vorzugsweise über 30 Minuten;
d) Thermische Behandlung des verdünnten Kulturmediums aus b) im Bioreaktor; vorzugsweise bei 85°C bis 90°C während 20 bis 30 Minuten;
e) Abkühlen des Kulturmediums aus d) bis zur Beimpfungstemperatur;
f) Beimpfung des Kulturmediums aus e) mit einer wirksamen Menge einer Mutterkultur wenigstens eines mikrobiellen Bakterien-Mikroorganismus oder einer Mischung von Mikroorganismen;
g) Entwicklung der Biomasse während einer Zeit, die für insgesamt 8 bis 10 Zellduplikationen des/der Mikroorganismus/en ausreicht, bis der pH des Kulturmediums spontan auf einen Wert von 4,9 bis 5,2 sinkt;
h) Abkühlen der Kultur aus g) auf eine Temperatur von 4°C bis 8°C.

3. Verfahren gemäß einem der Ansprüche 1 bis 2, wobei der Bakterienstamm *Streptococcus thermophilus* DSM 16506 ist.

4. Verwendung einer flüssigen Starterkultur, die nach dem Verfahren von einem der Ansprüche 1 bis 3 hergestellt wurde, als Starter zur Herstellung von industriellen Nahrungsmittelprodukten.

5. Verwendung gemäß Anspruch 4, wobei diese industriellen Nahrungsmittelprodukte ausgewählt werden aus der Gruppe bestehend aus: Milchprodukten, Käse, Joghurt, fermentierten Milchgetränken, Brot, Backwaren, Salamis, fermentierten Würsten, alkoholischen Getränken.

6. Verwendung gemäß Anspruch 5, wobei diese Produkte aus Milchprodukten ausgewählt werden.

7. Verwendung einer flüssigen Starterkultur, hergestellt nach dem Verfahren gemäß einem der Ansprüche 1 bis 3, zur direkten Beimpfung von Milch.

## Revendications

1. Procédé de préparation d'une culture de départ liquide comprenant au moins un micro-organisme choisi dans le groupe de souches microbiennes de l'espèce *Streptococcus thermophilus* ayant une concentration en cellules microbiennes dudit au moins un micro-organisme de plus de 1 x 10⁹ CFU/ml,
dans lequel ledit procédé comprend une étape dans laquelle un milieu de culture est ajouté avec une quantité efficace d'au moins un agent neutralisant basique contenant un mélange de carbonate de calcium et de monohydrogénophosphate de potassium,
dans lequel ledit agent de neutralisation est présent dans une quantité telle qu'il est possible de garder la valeur du pH dudit milieu au sein d'une plage donnée, durant la phase de croissance exponentielle de la biomasse microbienne dans ledit milieu,
dans lequel ladite plage de pH est de 5,0 à 5,7, et
dans lequel la concentration de la quantité totale de carbonate de potassium et de monohydrogénophosphate de potassium dans la culture est de 8 g/l à 20 g/l.

2. Procédé selon la revendication 1, comprenant les étapes suivantes :
a) l'addition à un milieu de culture d'une quantité d'au moins un agent neutralisant basique ;
b) la dissolution du milieu issu de a) dans un milieu liquide, de préférence l'eau ;
c) la décontamination du bioréacteur en faisant circuler de la vapeur ; de préférence pendant 30 minutes ;
d) le traitement thermique du milieu de culture dilué issu de b) dans le bioréacteur ; de préférence à 85 °C à 90 °C pendant 20 à 30 minutes ;
e) le refroidissement du milieu de culture issu de d) jusqu'à la température d'inoculation ;
f) l'inoculation du milieu de culture issu de e) avec une quantité efficace de la culture mère d'au moins un micro-organisme bactérien microbien, ou d'un mélange de micro-organismes ;
g) l'étape permettant à la biomasse de se développer pendant un temps suffisant pour qu'au total, 8 à 10 duplications cellulaires se déroulent, jusqu'à ce que le pH du milieu de culture chute spontanément jusqu'à une valeur de 4,9 à 5,2 ;
h) le refroidissement de la culture issue de g) jusqu'à une température de 4 °C à 8 °C.

3. Procédé selon l'une quelconque des revendications 1 à 2, dans lequel la souche bactérienne est *Streptococcus thermophilus* DSM 16506.

4. Utilisation d'une culture de départ liquide préparée selon le procédé de l'une quelconque des revendications 1 à 3 comme départ pour la préparation de produits alimentaires industriels.

5. Utilisation selon la revendication 4, dans laquelle lesdits produits alimentaires industriels sont choisis dans le groupe comprenant : des produits laitiers, des fromages, des yaourts, des laits fermentés, des pains, des produits cuits, des salamis, des saucisses fermentées, des boissons alcoolisées.

6. Utilisation selon la revendication 5, dans laquelle lesdits produits sont choisis parmi des produits laitiers.

7. Utilisation d'une culture de départ liquide préparée selon le procédé de l'une quelconque des revendications 1 à 3 pour une inoculation directe de lait.
